Europäisches Patentamt

(19) European Patent Office (11) Numéro de publication: **0 216 164**

Office européen des brevets **B1**

(12) FASCICULE DE BREVET EUROPÉEN

(45) Date de publication du fascicule du brevet: (51) Int. Cl. ⁵: **A 61 F 13/15,** B 31 D 1/04
21.03.90

(21) Numéro de dépôt: **86111717.4**

(22) Date de dépôt: **25.08.86**

(54) Procédé de fabrication en continu d'un matelas absorbant en forme de sablier pour une couche-culotte.

(30) Priorité: **27.08.85 FR 8512808**

(43) Date de publication de la demande:
**01.04.87 Bulletin 87/14**

(45) Mention de la délivrance du brevet:
**21.03.90 Bulletin 90/12**

(84) Etats contractants désignés:
**AT BE CH DE GB IT LI NL**

(56) Documents cité:
**US-A-2 122 417**

(73) Titulaire: **PEAUDOUCE**
**59, Rue de la Vignette**
**F-59126 Linselles (FR)**

(72) Inventeur: **Lucas, Gérard**
**Chemin de la Montagne**
**F-59117 Wervicq Sud (FR)**
Inventeur: **Dussaud, Jacques**
**26, Avenue du Maréchal Leclerc**
**F-59110 La Madeleine (FR)**
Inventeur: **Leroy, André**
**103, Rue St. Joseph**
**F-59166 Bousbecque (FR)**

(74) Mandataire: **Casalonga, Axel**
**BUREAU D.A. CASALONGA - JOSSE Morassistrasse 8**
**D-8000 München 5 (DE)**

LIBERGRAF, STOCKHOLM 1990

## Description

La présente invention se rapporte à un procédé de fabrication en continu de matelas absorbants en forme de sablier, c'est-à-dire ayant une forme générale rectangulaire avec deux échancrures latérales opposées, pour des couches-culottes.

Les matelas absorbants en forme de sablier comprennent, dans le sens de la longueur du matelas, deux zones d'extrémités et une zone intermédiaire d'entrejambe de largeur inférieure à celle des zones d'extrémités. Pour des raisons d'efficacité, il est désirable que ces matelas présentent, dans le sens de la largeur, au moins dans la zone d'entrejambe, une partie centrale d'épaisseur supérieure à celle des parties latérales.

On connaît deux modes de réalisation de matelas absorbants de ce type.

Suivant un premier mode de réalisation, un matelas est constitué d'un tronçon rectangulaire de bande de matériau absorbant, dans chacun des deux bords latéraux opposés duquel sont pratiquées deux incisions, la partie délimitée entre ces deux incisions étant rabattue en direction du bord latéral opposé, de manière que les deux rabats opposés forment une double épaisseur dans la zone d'entrejambe du matelas. Ce rabattement n'est pas sans présenter des difficultés à des cadences élevées de fabrication. Par ailleurs, ce rabattement doit se faire avec une précision élevée de manière que les bords libres des rabats opposés soient contigüs. En effet, dans le cas contraire, ces bords peuvent, soit être espacés l'un de l'autre, soit se chevaucher, ce qui altère la fonction du matelas absorbant ainsi que l'aspect de la couche-culotte.

Selon un autre mode de réalisation connu, un matelas absorbant est formé par superposition d'un tronçon rectangulaire de matériau absorbant, ayant une largeur inférieure ou égale à la largeur d'entrejambe du matelas absorbant complet, à un tronçon de matériau absorbant en forme de sablier. Pour la fabrication en continu de tels matelas, on découpe une bande de matériau absorbant suivant une ligne de découpe longitudinale, rectiligne, en une première bande partielle relativement étroite et en une seconde bande partielle plus large, on découpe ensuite dans la bande partielle plus large des échancrures diamétralement opposées et on pose la bande partielle plus étroite sur la bande partielle plus large avant de découper la bande composite ainsi formée transversalement dans les zones comprises entre les échancrures successives. Le principal inconvénient de ce procédé qui permet également d'obtenir des matelas absorbants ayant une double épaisseur dans la zone d'entrejambe réside dans les chutes dues aux découpes pratiquées dans la bande partielle plus large en vue de la réalisation des échancrures.

Il s'est avéré que pour des raisons d'efficacité, il serait désirable de disposer d'une épaisseur encore accrue, à savoir une triple épaisseur, dans la zone d'entrejambe. Ce résultat pourrait, en principe, être obtenu par la combinaison des deux modes de réalisation ci-dessus, à savoir par la découpe d'une bande de matériau absorbant suivant une ligne de découpe longitudinale rectiligne en une première bande partielle étroite et en une seconde bande partielle plus large, selon le second mode de réalisation, par transformation de la bande plus large en une bande à rabats suivant le premier mode de réalisation, et par la superposition de la bande plus étroite à cette bande à rabats, en vue d'obtenir ainsi une triple épaisseur dans la zone d'entrejambe. Toutefois, un tel procédé conserverait toujours les inconvénients du premier mode de réalisation, à savoir notamment la faible cadence de fabrication.

Il est par ailleurs connu par le document US-A-2 122 417 de réaliser des culottes en forme de sablier, en tissu, papier ou autres matériaux absorbants, chaque culotte comportant, dans le sens de sa longueur, deux zones d'extrémité et une zone intermédiaire d'entrejambe de largeur inférieure à celle des zones d'extrémité, et présentant, dans la partie médiane de sa largeur, notamment dans la zone d'entrejambe une épaisseur triple de celle des parties latérales des zones d'extrémité. Ces culottes sont réalisées à partir d'une pièce rectangulaire de tissu, papier ou analogue, présentant, dans deux zones d'extrémité, des incisions partant des bords transversaux, cette pièce étant soumise à un double pliage autour de deux lignes longitudinales s'étendant entre les extrémités intérieures desdites incisions, de manière à obtenir une triple épaisseur de matériau absorbant au moins sur la partie médiane de la largeur de la zone d'entrejambe et une simple épaisseur de matériau absorbant dans les parties latérales des zones d'extrémité. Il est par ailleurs prévu, suivant ce document, de fabriquer les culottes à partir d'une bande continue de matériau absorbant de telle manière que lesdites lignes de pliage s'étendent transversalement à la longueur de la bande. De ce fait, il est nécessaire, après avoir pratiqué les incisions dans la bande, de découper cette dernière en tronçons successifs avant de pouvoir procéder au double pliage sur chacun des tronçons de la bande de matériau absorbant. Cela complique la fabrication, empêche une fabrication réellement en continu et limite la cadence de fabrication.

La présente invention a pour objet un procédé de fabrication en continu particulièrement simple et rapide de matelas absorbants en forme de sablier comportant, dans la partie médiane de leur longueur, notamment dans la zone d'entrejambe, une épaisseur triple de celle des parties latérales des zones d'extrémité.

Suivant un premier mode de réalisation du procédé conforme à l'invention, tel que revendiqué, on pratique successivement, dans une bande continue de matériau absorbant, de largeur supérieure à la largeur maximale des matelas à fabriquer, à des intervalles correspondant à la longueur des matelas à fabriquer, deux incisions opposées symétriques par rapport à la ligne médiane longitudinale de la bande et comprenant, dans le sens de la longueur de la bande, deux parties allant en divergeant suivies de deux parties longitudinales suivies de deux parties allant en convergeant. On plie les deux parties de la bande située entre chaque incision et les lignes longitudinales reliant entre elles les incisions successives, d'une part, et le bord longitudinal correspondant de la bande, d'autre part, autour desdites lignes longitudinales l'une sur l'autre et sur la partie de la bande située entre lesdites lignes. Ensuite, on coupe la bande pliée transversalement à des intervalles correspondant à la longueur des matelas à fabriquer, à l'endroit où la distance réciproque des deux incisions opposées est maximale.

Selon un second mode de réalisation du procédé conforme à l'invention, tel que revendiqué, on pratique successivement, dans une bande continue de matériau absorbant, de largeur supérieure à la largeur maximale desmatelas à fabriquer, à des intervalles correspondant à la longueur des matelas à fabriquer, deux incisions symétriques par rapport à la ligne médiane longitudinale de la bande et comprenant, dans le sens de la longueur de la bande, deux parties allant en convergeant suivies d'une partie médiane longitudinale suivies de deux parties allant en divergeant. On plie les deux parties de la bande situées entre chaque incision et les lignes longitudinales reliant entre elles les incisions successives, d'une part, et le bord longitudinal correspondant de la bande, d'autre part, autour desdites lignes longitudinales l'une sur l'autre et sur la partie de la bande située entre lesdites lignes. Enfin, on coupe la bande pliée transversalement à des intervalles correspondant à la longueur des matelas à fabriquer, à l'endroit où la distance réciproque des deux incisions opposées est maximale.

Dans le premier cas, il est possible de plier les deux parties latérales l'une par-dessus l'autre sur la partie médiane. Par contre, dans le second cas, il est avantageux de plier la bande en Z, c'est-à-dire d'abord la partie médiane avec l'une des parties latérales dans un sens sur l'autre partie latérale et de plier ensuite cette autre partie dans le sens contraire sur la partie médiane superposée à la première partie latérale.

En se référant aux dessins annexés, on va décrire ci-après plus en détail deux modes de réalisation de l'objet de l'invention; sur les dessins:

- la figure 1 est une vue en plan d'un premier mode de réalisation d'un matelas absorbant fabriqué suivant le procédé conforme à l'invention;
- la figure 2 est une coupe suivant II – II de la figure 1;
- les figures 3 et représentent à plus petite échelle deux étapes du procédé de fabrication en continu de matelas absorbants suivant les figures 1 et 2;
- la figure 5 est une vue en plan d'un second mode de réalisation d'un matelas absorbant fabriqué suivant le procédé conforme à l'invention;

- la figure 6 est une coupe suivant VI – VI de la figure 5;
- les figures 7 et 8 représentent à plus petite échelle deux étapes du procédé de fabrication en continu de matelas absorbants suivant les figures 5 et 6.

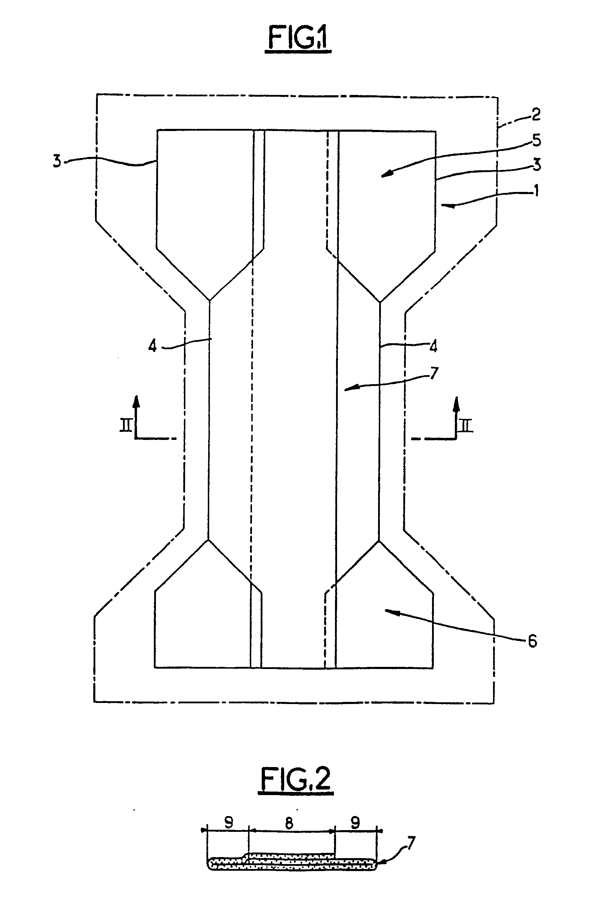

Selon la figure 1, un matelas absorbant 1 destiné à être incorporé à une couche-culotte 2 dont seul le contour est indiqué en traits mixtes sur la figure 1 présente, tout comme la couche-culotte 2, une forme en sablier, connue en soi. Cette forme en sablier est due à la présence, dans chacun des deux bords longitudinaux opposés 3 du matelas 1, d'une découpe 4 en forme d'échancrure trapézoïdale. Le matelas absorbant 1 comprend ainsi une partie d'extrémité arrière 5, une partie d'extrémité avant 6 et une partie intermédiaire d'entrejambe 7 située entre les deux échancrures 4. On reconnaît sur la figure 1 que la partie d'extrémité arrière 5 peut, de façon connue en soi, être quelque peu plus haute que la partie d'extrémité avant 6. Pour la fermeture de la couche-culotte, la partie d'extrémité 1 arrière peut, de façon connue, être munie d'attaches adhésives, non représentées.

Selon les figures 1 et 2, le matelas absorbant 1 formé à partir d'une pièce rectangulaire de matériau absorbant plat, présente une triple épaisseur dans la partie médiane 8 de la largeur de la zone d'entrejambe 7 et une double épaisseur dans les deux parties latérales 9 de la zone d'entrejambe 7. Dans les deux zones d'extrémités 5 et 6, le matelas présente également une triple épaisseur dans la partie médiane et une simple épaisseur dans les parties latérales.

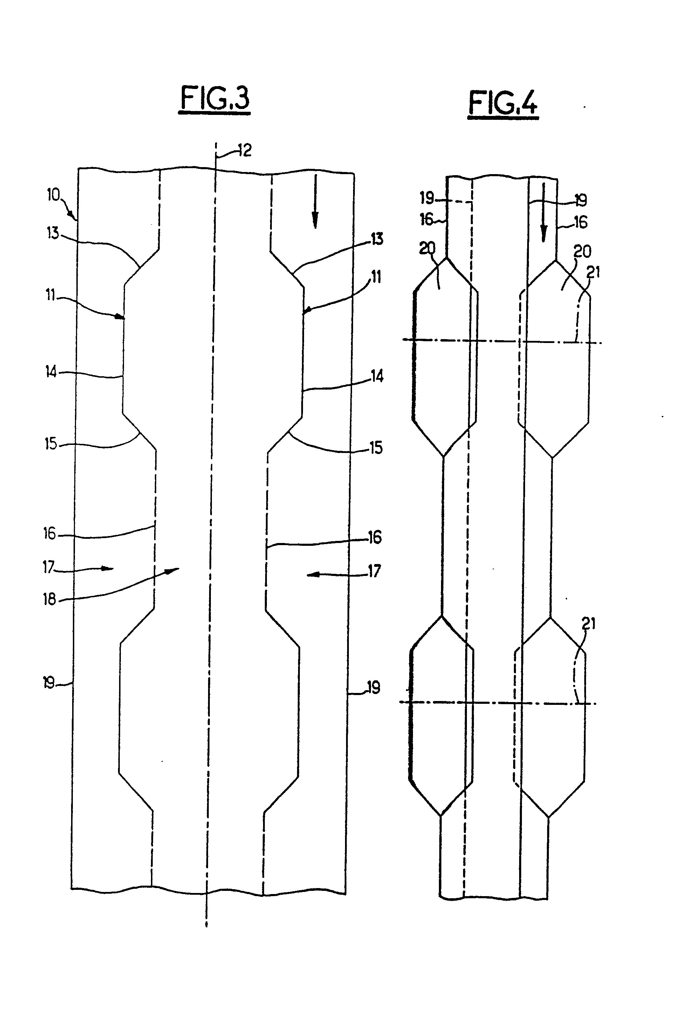

Pour fabriquer en continu des matelas absorbants suivant les figures 1 et 2, on pratique, selon la figure 3, dans une bande 10 de matériau absorbant, en cours de défilement de la bande, successivement, à des intervalles correspondant à la longueur des matelas à fabriquer, deux incisions 11 opposées, symétriques par rapport à la ligne médiane longitudinale 12 de la bande 10. Les deux incisions 11 comprennent, dans le sens de défilement de la bande 10, deux segments de droite 13 divergents, suivis de deux segments de droite 14 longitudinaux eux-mêmes suivis de deux segments de droite 15 convergents. En plus de ces incisions 11, on ménage dans la bande 10, deux lignes de pliage 16 longitudinales, s'étendant entre les extrémités des incisions 11 successives. Il y a lieu de noter que la distance entre les segments de droite longitudinaux 14 des incisions opposées 11 est égale à la largeur maximale des matelas absorbants 1 dans les zones d'extrémités 5 et 6, tandis que la distance entre les lignes de pliage opposées 16 est égale à la largeur du matelas absorbant 1 dans la zone d'entrejambe 7 (voir figures 1 et 2). Par conséquent, la distance séparant chaque ligne de pliage longitudinale 16 du bord longitudinal 19 correspondant de la bande 10, est inférieure à la distance séparant les deux lignes de pliage 16 opposées.

Après avoir ménagé les incisions 11 et les lignes de pliage 16, on rabat en continu vers l'intérieur, autour des lignes de pliage 16, successivement l'une et ensuite l'autre des deux parties latérales 17 délimitées dans la bande 10 par les lignes de pliage 16 et les incisions 11, d'une part, et les bords longitudinaux correspondants 19 de la bande 10, sur la partie centrale 18 délimitée entre les lignes de pliage 16 et les incisions 11, d'autre part, pour former une bande telle qu'illustrée par la figure 4, de manière que les deux parties latérales 17 se chevauchent sur la partie centrale 18. La bande ainsi pliée comporte par intervalles des oreilles latérales 20 opposées de simple épaisseur, dues aux incisions 11.

Il suffit ensuite de découper la bande pliée par des coupes transversales 21, au droit des oreilles 20, à des intervalles correspondant à la longueur des matelas absorbants à fabriquer. On remarque sur la figure 4 que les lignes de découpe transversales 21 ne passent pas par le milieu de la longueur des oreilles 20, ce qui permet d'obtenir des matelas absorbants ayant deux zones d'extrémités de hauteurs différentes, comme représenté sur la figure 1.

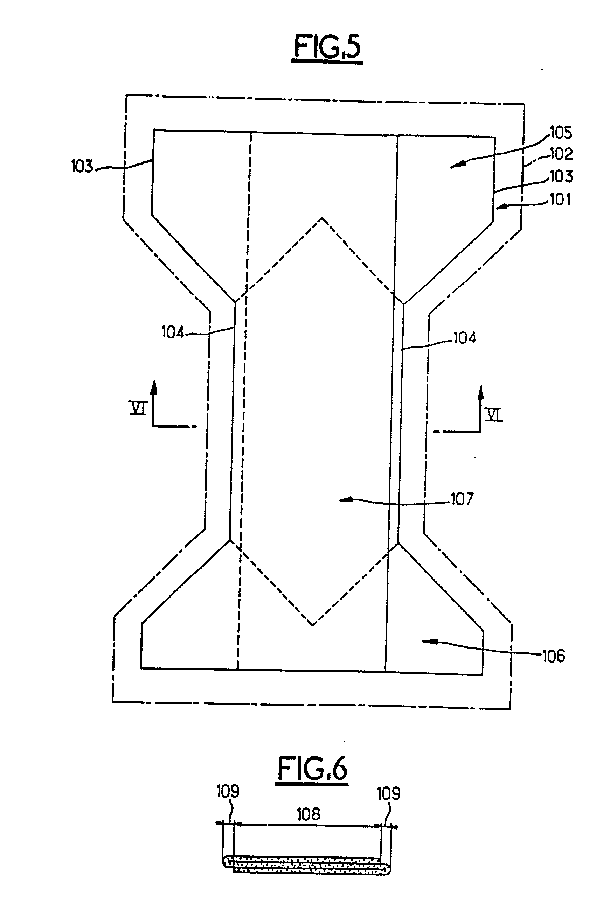

Le matelas absorbant 101 selon la figure 5 présente la même forme générale que le matelas 1 de la figure 1. Ce matelas destiné à une couche-culotte 102 indiquée schématiquement par son contour, est également en forme de sablier et comporte, dans chacun de ses deux bords longitudinaux opposés 103, une découpe 104 en forme d'échancrure trapézoïdale, ce qui définit une zone d'extrémité arrière 105, une zone d'extrémité avant 106 et une zone intermédiaire d'entrejambe 107 située entre les deux échancrures 104.

On retrouve par ailleurs, comme le montrent les figures 5 et 6, dans le sens de la largeur de la zone d'entrejambe 107, une partie médiane 108 de triple épaisseur et deux parties latérales 109 de double épaisseur.

Le matelas 101 diffère principalement du matelas 1 de la figure 1 par le fait que la partie 108 de triple épaisseur ne s'étend pas sur toute la longueur du matelas 101, mais est limitée sensiblement à la zone d'entrejambe 107, alors que les zones d'extrémités 105 et 106 sont à double épaisseur dans leur partie médiane et à simple épaisseur dans leurs parties latérales.

Pour fabriquer en continu des matelas absorbants suivant les figures 5 et 6, on ménage dans une bande 110 de matériau absorbant, d'une largeur supérieure à la largeur des matelas à fabriquer, successivement à des intervalles correspondant à la longueur des matelas à fabriquer, une entaille 111 symétrique par rapport à l'axe médian longitudinal 112 de la bande 110. Chaque entaille 111 comprend dans le sens de défilement de la bande 110 deux segments de droite 113 convergeant sur la ligne 112, suivis d'un segment de droite unique 114 longitudinal, lui-même suivi de deux segments de droite 115 divergents. On pratique également dans la bande 110, entre les extrémités libres des segments de droite 113 et 115 des incisions 111 successives, des lignes longitudinales de pliage 116. De cette manière, la bande 110 est subdivisée, entre deux incisions successives 111, par les lignes de pliage 116, en deux parties latérales 117 situées respectivement entre une ligne de pliage 116 et le bord longitudinal 119 correspondant de la bande 110, et une partie médiane 118 située entre les deux lignes de pliage 116.

La bande 110 subit ensuite en continu un pliage autour des lignes 116, à savoir un premier pliage à la fois de la partie médiane 118 et d'une partie latérale 117, autour de la ligne de pliage 116 opposée, sur l'autre partie latérale 117, puis un second pliage de la première partie latérale 117 autour de l'autre ligne de pliage 116 sur la partie médiane 118 (pliage en Z), comme cela apparaît surtout sur la figure 6. Dans le mode de réalisation représenté, ce double pliage comprend d'abord un pliage vers la gauche, sur la partie latérale 117 de gauche, de la partie médiane 118 et de la partie latérale 117 de droite, puis un pliage vers la droite de la seule partie latérale 117 initialement de droite.

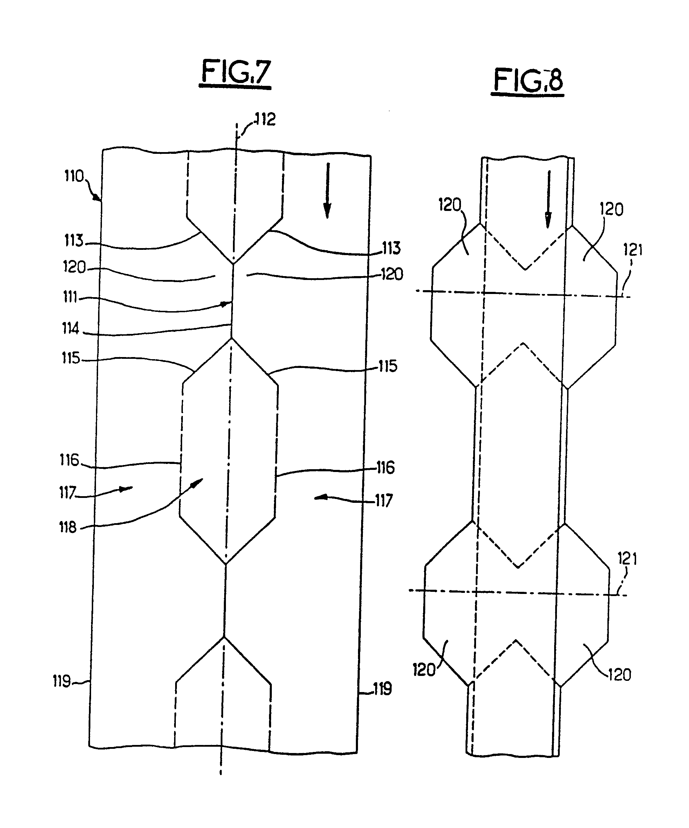

L'avantage particulier de ce mode de pliage consiste dans le fait qu'il peut être effectué de façon simple alors que la bande avance sur un support, sans que ce pliage ne soit gêné par les "oreilles" 120 qui, du fait de la forme des incisions 111, dépassent les parties latérales 117 au-delà des lignes de pliage 120 et qui, après le double pliage, font saillie en simple épaisseur sur la partie médiane à double et triple épaisseur de la bande (voir figure 8).

Il suffit ensuite de couper la bande suivait une ligne de coupe transversale 121 à des intervalles correspondant à la longueur des matelas absorbants à fabriquer, à l'endroit des oreilles 120, pour obtenir des matelas absorbants individuels. Comme indiqué sur la figure 8, le découpage en 121 ne s'effectue pas au milieu de la longueur des oreilles 120, afin d'obtenir ainsi, comme illustre sur la figure 5, un matelas absorbant ayant des zones d'extrémités de hauteurs différentes.

Il va de soi que les modes de réalisation décrits ci-dessus et illustrés par les dessins annexés n'ont été donnés qu'à titre d'exemples non limitatifs et que de nombreuses modifications et variantes sont possibles dans le cadre de l'invention.

Ainsi, les incisions 11 et 111, au lieu d'être formées de segments de droite, pourraient également comprendre des segments incurvés, ce qui donnerait aux matelas absorbants 1, 101, des échancrures de forme curviligne.

Par ailleurs, les zones d'entrejambe 7, 107, pourraient être à triple épaisseur sur toute leur largeur.

Le matelas absorbant peut être fabriqué à partir de n'importe quel matériau absorbant en forme de bande, par exemple de la ouate ou mousse de cellulose ("fluff") avec ou sans matériau superabsorbant incorporé et avec ou sans matériau de doublage tel que papier, etc...

## Revendications

1. Procédé de fabrication en continu de matelas absorbants en forme de sablier, ayant une forme générale rectangulaire avec deux échancrures latérales opposées, pour une couche-culotte, chaque matelas comprenant, dans le sens de sa longueur, deux zones d'extrémité et une zone intermédiaire d'entrejambe de largeur inférieure à celle des zones d'extrémité, et présentant dans la partie médiane de sa largeur, notamment dans la zone d'entrejambe, une épaisseur triple de celle des parties latérales des zones d'extrémité, caractérisé par le fait qu'on pratique successivement, dans une bande de matériau absorbant (10) de largeur supérieure à la largeur maximale des matelas à fabriquer, à des intervalles correspondant à la longueur des matelas à fabriquer, deux incisions opposées (11) symétriques par rapport à la ligne médiane longitudinale (12) de la bande et comprenant, dans le sens de la longueur de la bande, deux parties (13) allant en divergeant, suivies de deux parties (15) allant en convergeant, qu'on plie les deux parties latérales (17) de la bande (10), comprises entre chaque incision (11) et les lignes longitudinales (16) reliant entre elles les incisions successives d'une part, et le bord longitudinal correspondant (19) de la bande (10), d'autre part, autour desdites lignes longitudinales, l'une sur l'autre et sur la partie médiane (18) de la bande situées entre lesdites lignes (16), et qu'on coupe la bande ainsi pliée transversalement, à des intervalles correspondant à la longueur des matelas absorbants à fabriquer, à l'endroit où la distance séparant les incisions opposées (11) est maximale.

2. Procédé de fabrication en continu de matelas absorbants en forme de sablier, ayant une forme générale rectangulaire avec deux échancrures latérales opposées, pour une couche-culotte, chaque matelas comprenant, dans le sens de sa longueur, deux zones d'extrémité et une zone intermédiaire d'entrejambe de largeur inférieure à celle des zones d'extrémité, et présentant dans la partie médiane de sa largeur, notamment dans la zone d'entrejambe, une épaisseur triple de celle des parties latérales des zones d'extrémité, caractérisé par le fait qu'on pratique successivement, dans une bande de matériau absorbant (110) de largeur supérieur à la largeur maximale des matelas à fabriquer, à des intervalles correspondant la longueur des matelas à fabriquer, deux incisions (111) symétriques par rapport à la ligne médiane longitudinale (112) de la bande et comprenant, dans le sens de la longueur, de la bande, deux parties (113) allant en convergeant, suivies de deux parties (115) allant en divergeant, qu'on plie les deux parties latérales (117) de la bande (110), comprises entre les incisions (111) et les lignes longitudinales (116) reliant entre elles les incisions successives, d'une part, et le bord longitudinal correspondant (119) de la bande (110), d'autre part, autour desdites lignes longitudinales, l'une sur l'autre et sur la partie médiane (118) de la bande, et qu'on coupe la bande ainsi pliée transversalement à des intervalles correspondant à la longueur des matelas absorbants à fabriquer, à l'endroit où la distance séparant les incisions opposées est maximale.

3. Procédé suivant la revendication 2, caractérisé par le fait qu'on plie la bande (110) en Z autour des lignes longitudinales (116).

4. Procédé suivant l'une quelconque des revendications 1 à 3, caractérisé par le fait qu'on ménage des lignes de pliage (16, 116) longitudinales dans le bande (10, 110) avant de procéder au pliage.

## Patentansprüche

1. Verfahren zum kontinuierlichen Herstellen von saugfähigen Kissen in Form einer Sanduhr, die eine im wesentlichen rechteckige Form mit zwei seitlichen, gegenüberliegenden Aussparungen hat, für ein Windelhöschen, wobei jedes Kissen in seiner Längsrichtung zwei Endzonen und eine Zwischenzone als Schrittbereich mit gegenüber den Endzonen verringerter Breite aufweist und im Mittelteil seiner Breite, insbesondere im Schrittbereich, eine gegenüber den Seitenteilen der Endzonen dreifache Dicke aufweist, dadurch gekennzeichnet, daß man nacheinander in einem Band aus saugfähigem Werkstoff (10) mit einer Breite, die größer ist als die größte Breite des herzustellenden Kissens, in Abständen, die der Länge der herzustellenden Kissen entsprechen, zwei gegenüberliegende, zur Längsmittellinie (12) des Bandes symmetrische Einschnitte (11) erzeugt, die in Längsrichtung des Bandes zwei divergierend verlaufende Abschnitte (13), gefolgt von zwei konvergierend verlaufenden Abschnitten (15) besitzen, daß man die beiden Seitenteile (17) des Bandes (10), die zwischen jedem Einschnitt (11) und den Längslinien (16), welche die aufeinanderfolgenden Einschnitte miteinander verbinden, einerseits und dem entsprechenden Längsrand (19) des Bandes (10) anderseits angeordnet sind, um die genannten Längslinien aufeinander und auf den zwischen den Linien (16) angeordneten Mittelteil (18) des Bandes faltet, und daß man das so gefaltete Band in Abständen, die der Länge des herzustellenden saugfähigen Kissens entsprechen, an der Stelle quer durchschneidet, wo der Abstand zwischen den gegenüberliegenden Einschnitten (11) am größten ist.

2. Verfahren zum kontinuierlichen Herstellen von saugfähigen Kissen in Form einer Sanduhr, die eine im wesentlichen rechteckige Form mit zwei seitlichen, gegenüberliegenden . Aussparungen hat für ein Windelhöschen, wobei jedes Kissen in seiner Längsrichtung zwei Endzonen und eine Zwischenzone als Schrittbereich mit gegenüber den Endzonen verringerter Breite aufweist und im Mittelbereich seiner Breite, insbesondere im Schrittbereich, eine gegenüber den Seitenteilen der Endzonen dreifache Dicke aufweist, dadurch gekennzeichnet, daß man nacheinander in einem Band aus saugfähigen Werkstoff (110) mit einer Breite, die grösser ist als die größte Breite des herzustellenden Kissens, in Abständen, die der Länge des herzustellenden Kissens entsprechen, zwei zur Längsmittellinie (112) des Bandes symmetrische Einschnitte (111) erzeugt, die in Längsrichtung des Bandes zwei konvergierend verlaufende Abschnitte (113), gefolgt von zwei divergierend verlaufenden Teilen (115) besitzen, daß man die beiden Seitenteile (117) des Bandes (110), die zwischen den Einschnitten (111) und den Längslinien (116), welche die aufeinanderfolgenden Einschnitte miteinander verbinden, einerseits und dem entsprechenden Längsrand (119) des Bandes (110) anderseits liegen, um diese Längslinien aufeinander und auf den Mittelteil (118) des Bandes umfaltet und daß man das so gefaltete Band in Abständen, die der Länge des herzustellenden saugfähigen Kissens entsprechen, an der Stelle quer durchschneidet, wo der Abstand zwischen den gegenüberliegenden Einschnitten am grössten ist.

3. Verfahren nach Anspruch 2, dadurch gekennzeichnet, daß man das Band (110) Z-förmig um die Längslinien (116) faltet.

4. Verfahren nach einem der Ansprüche 1 bis 3, dadurch gekennzeichnet, daß man im Band (10, 110) längslaufende Faltlinien (16, 116) erzeugt, bevor das Falten ausgeführt wird.

## Claims

1. A method for the continuous production of hour glass-shaped absorbent pads for a nappy, which pads have a generally rectangular shape with two opposed lateral recesses, each pad comprising in the direction of its length, two end zones and an intermediate crutch zone with a width smaller than that of the end zones and having, in the median portion of its width, in particular in the crutch zone, a thickness three times that of the lateral portions of the end zones, characterized in that a web of absorbent material (10) with a width exceeding the maximum width or the pads to be produced, is provided successively at intervals corresponding to the length of the pads to be produced, with two opposed cuts (11) symmetrical in relation to the longitudinal median line (12) of the web and comprising in the direction of the web length, two divergent portions (13) followed by two converging portions (15), in that the two lateral portions (17) of the web (10) comprised between each cut (11) and the longitudinal lines (16) interconnecting the successive cuts on the one hand, and the corresponding longitudinal edge (19) of the web (10) on the other hand, are folded around the said longitudinal lines, one onto the other and onto the median portion (18) of the web located between the said lines (16), and in that the web which is thus folded, is transversely cut at intervals corresponding to the length of the absorbent pads to be produced, at the point where the distance separating the opposed cuts (11) is at its greatest.

2. A method for the continuous production of hour glass-shaped absorbent pads for a nappy, which pads have a generally rectangular shape with two opposed lateral recesses, each pad comprising in the direction of its length, two end zones and an intermediate crutch zone with a width smaller than that of the end zones and having, in the median portion of its width, in particular in the crutch zone, a thickness three times that of the lateral portions of the end zones, characterized in that a web of absorbent material (110) with a width exceeding the maximum width of the pads to be produced, is provided successively, at intervals corresponding to the length of the pads to be produced, with two cuts (111) symmetrical in relation to the longitudinal median line (112) of the web and comprising in the direction of the web length two converging portions (113), followed by two divergent portions (115), in that the two lateral portions (117) of the web (110) comprised between the cuts (111) and the longitudinal lines (116) interconnecting the successive cuts on the one hand, and the corresponding longitudinal edge (119) of the web (110) on the other hand, are folded around the said longitudinal lines, one onto the other and onto the median portion (118) of the web, and in that the web thus folded, is transversely cut at intervals corresponding to the length of the absorbent pads to be produced, at the point where the distance separating the opposed cuts is at its greatest.

3. A method according to claim 2, characterized in that the web (110) is folded in a zig-zag configuration around the longitudinal lines (116).

4. A method according to any one of claims 1 to 3, characterized in that longitudinal fold lines (16, 116) are arranged in the web (10, 110) before proceeding with the folding.

## FIG.1

## FIG.2

## FIG.3

## FIG.4

## FIG.5

## FIG.6

## FIG.7

## FIG.8